# EUROPÄISCHE PATENTSCHRIFT

(11) **EP 1 150 682 B1**
(45) Veröffentlichungstag und Bekanntmachung des Hinweises auf die Patenterteilung: **17.08.2005**
(21) Anmeldenummer: 00914075.7
(22) Anmeldetag: 09.02.2000
(51) Int. Cl.: A61K 31/519, A61P 25/30

(54) **UBICHINON Qn ZUR BEHANDLUNG VON MIGRÄNESCHMERZEN**
UBIQUINONE Qn FOR THE TREATMENT OF MIGRAINE PAINS
COENZYME Qn UTILISE POUR TRAITER LA DOULEUR DE LA MIGRAINE

(30) Priorität: 11.02.1999 DE 19905879
(43) Veröffentlichungstag der Anmeldung: 07.11.2001
(73) Patentinhaber: MSE Pharmazeutika GmbH, 61348 Bad Homburg (DE)
(72) Erfinder: ENZMANN, Franz, D-61348 Bad Homburg v.d.H. (DE)
(74) Vertreter: Werner, Hans-Karsten, Dr.Dipl.-Chem.
(86) Internationale Anmeldenummer: PCT/EP2000/001011
(87) Internationale Veröffentlichungsnummer: WO 2000/047192

(56) Entgegenhaltungen:
- WO-A-00/47185
- WO-A-98/35658
- DATABASE WPI Section Ch, Week 198720 Derwent Publications Ltd., London, GB; Class B05, AN 1987-139143 XP002155697 & JP 62 077318 A (EISAI CO LTD), 9. April 1987 (1987-04-09)
- PATENT ABSTRACTS OF JAPAN vol. 1996, no. 02, 29. Februar 1996 (1996-02-29) & JP 07 278002 A (TAISHO PHARMACEUT CO LTD), 24. Oktober 1995 (1995-10-24)
- PATENT ABSTRACTS OF JAPAN vol. 013, no. 427 (C-639), 22. September 1989 (1989-09-22) & JP 01 165522 A (M S C:KK), 29. Juni 1989 (1989-06-29)
- MATSUMURA T ET AL: "EVIDENCE FOR ENHANCED TREATMENT OF PERIODONTAL DISEASE BY THERAPY WITH COENZYME Q" INTERNATIONAL JOURNAL FOR VITAMIN & NUTRITION RESEARCH,HOGREFE AND HUBER, BERNE,CH, Bd. 43, Nr. 4, April 1973 (1973-04), Seiten 537-548, XP000965181 ISSN: 0300-9831
- OSTMAN B ET AL: "THE EFFECT OF VITAMIN E AND UBIQUINONE-10 SUPLLEMENTATION ON PHYSICAL PERFORMANCE AND MUSCLE METABOLISM" CLINICAL SCIENCE,BIOCHEMICAL SOCIETY AND THE MEDICAL RESEARCH SOCIETY, LONDON,,GB, Bd. 87, Nr. SUPPLEMENT, 1994, Seiten 80-81, XP000965218 ISSN: 0143-5221
- ORTEGA R ET AL: "Muscle pain in a young cyclist." MEDICINE AND SCIENCE IN SPORTS AND EXERCISE, Bd. 25, Nr. 5 SUPPL., 1993, Seite S65 XP002155696 40th Annual Meeting of the American College of Sports Medicine on Medicine and Science in Sports and Exercise;Seattle, Washington, USA; June 2-5, 1993 ISSN: 0195-9131
- LINDH G ET AL: "PLASMA NUTRIENTS IN JOINT AND MUSCLE PAIN PATIENTS BEFORE AND AFTERNUTRITIONAL THERAPY" J. NUTR. ENV. MED., Bd. 7, Nr. 1, 1997, Seiten 15-16, XP000965078

## Beschreibung

Ubichinone sind prenylierte Chinone und in der Tier- und Pflanzenwelt weit verbreitet. Es handelt sich um Derivate von 2,3-Dimethoxy-5-methyl-1,4-benzochinon, die in Sechsstellung linear verknüpfte Isopreneinheiten aufweisen. Je nach Anzahl der Isopreneinheiten werden die Ubichinone als Q-1, Q-2, Q-3 usw. bezeichnet. Bei den meisten Säugetieren einschließlich des Menschen überwiegt Q-10 (2,3-Dimethoxy-5-methyl-6-decaprenyl-1,4-benzochinon). Ubichinone dienen als Elektronenüberträger in der Atmungskette, sie sind beteiligt an der zyklischen Oxidation und Reduktion von Substraten im Zitronensäurezyklus. Ubichinone Qₙ sind Voraussetzung für die Energieversorgung aller Zellen. Der oxidative Stress, der unter anderem durch hohen Sauerstoffverbrauch entsteht, löst Schäden an den Membranen von Mitchondrien und Zellen aus, die zu akuten oder degenerativen Störungen des Nervensystems führen. Das Nervensystem hat einen sehr hohen Energiebedarf für die Signalübertragung durch Membranpotentialaufbau, Ionenkanalsteuerung sowie durch Neuropeptid- und Neurotransmitter-Vesikelbildung.

Ubichinon Q-10 (auch Coenzym Q-10 genannt) wurde bisher in der Therapie von Herzerkrankungen verwendet.

Überraschenderweise wurde nun gefunden, dass Ubichinon Qₙ und Ubichinon Qₙ-Vorstufen zur Behandlung von Schmerzen verwendet werden können. Sie können somit auch eingesetzt werden in Verfahren zur Herstellung von Mitteln zur Behandlung von Schmerzen.

Mit Ubichinon Qₙ-Vorstufen werden Verbindungen bezeichnet, die im Körper zu Ubichinon Qₙ umgewandelt werden. Dies sind zum einen die Ubihydrochinone, die mit den Ubichinonen im Gleichgewicht stehen sowie einfache Ester der Ubihydrochinone mit kurzkettigen Carbonsäuren mit 1-10 C-Atomen, beispielsweise Essigsäure-, Propionsäure- oder Buttersäureester. Diese Vorstufen werden nach der Applikation in die entsprechenden Ubichinone umgewandelt.

Bevorzugt wird Ubichinon Q-10 verwendet, da dies das Haupt-Ubichinon beim Menschen ist.

Es lassen sich insbesondere Schmerzen behandeln, die durch Störung der Reizleitung in den Nerven verursacht werden und/oder in unangemessenem Verhältnis zur äußeren Ursache stehen. Dies sind Schmerzen, bei denen entweder kein äußerer Anlass besteht oder bei einem nur geringen Anlass und oxidativen Stresszuständen der Nerven ein überhöhtes Signal entsteht.

Es lassen sich die erfindungsgemäß zu verwendenden Substanzen zur Behandlung von Schmerzen einsetzen, die durch Migräne, verursacht werden. Die Behandlung kann durch Applikation in oraler, parenteraler, lokaler, inhalativer oder intranasaler Form erfolgen. Die Art der Applikation muss dabei auf den zu behandelnden Schmerzzustand abgestimmt werden. So hat es sich beispielsweise gezeigt, daß Migräne auch mit hohen Dosen Q-10 bei oraler Applikation nur sehr eingeschränkt behandelt werden kann. Bei Anwendung in Form von Mund- oder Nasensprays genügen jedoch schon kleine Mengen zur schnellen und effektiven Beseitigung von Migräneschmerzen. Gute Ergebnisse wurden bereits bei Dosen von etwa 20 mg erzielt.

Entscheidend ist, dass genügende Mengen der Ubichinone bzw. der Ubichinon-Vorstufen an den Ort kommen, an dem die durch Störungen der Signalübertragung des Nervensystems verursachten Schmerzen ihren Ursprung haben.

Durch Kombination mit Lung Surfactant Factor (Pulmonary Surfactant Factor) wie in der WO 08/35660 beschrieben, kann dieser Effekt noch verstärkt werden.

Aufgrund der geringen Dosis, die bereits effektiv ist, um Migräneschmerzen zu behandeln, wird erfindungsgemäß das Ubichinon Qₙ oder seine Vorstufen bevorzugt in Form eines Sprays, vorzugsweise eines Nasensprays verwendet.

Prinzipiell kann die Einzeldosis bis zu 1.000 mg betragen.

Ubichinone sind lipophile Substanzen, die in Wasser praktisch nicht löslich sind. Besonders hohe Effektivität wurde jedoch gefunden, wenn das Ubichinon Qₙ oder seine Vorstufen in einer wässrigen Dispersion vorliegen. Wässrige kolloidale Dispersionen sind besonders bevorzugt. Die Herstellung entsprechender Dispersionen ist in der WO 95/05164 sowie in der damit verbundenen DE-A-43 27 063 beschrieben.

## Patentansprüche

1. Verwendung von Ubichinon Qₙ oder Ubichinon Qₙ-Vorstufen zur Herstellung eines Arzneimittels zur Behandlung von Schmerzen, die durch Migräne verursacht werden.

2. Verwendung nach Anspruch 1, **dadurch gekennzeichnet, dass** das Ubichinon Qₙ Ubichinon Q₁₀ ist.

3. Verwendung nach einem der Ansprüche 1 bis 2, **dadurch gekennzeichnet, dass** die Behandlung durch Applikation in oraler, parenteraler, lokaler, inhalativer oder intranasaler Form erfolgt.

4. Verwendung nach Anspruch 3, **dadurch gekennzeichnet, dass** die Applikation in Form eines Sprays erfolgt.

5. Verwendung nach Anspruch 4, **dadurch gekennzeichnet, dass** die Applikation in Form eines Nasensprays erfolgt.

6. Verwendung nach einem der Ansprüche 1 bis 5, **dadurch gekennzeichnet, dass** das Ubichinon Qₙ oder die Ubichinon Qₙ-Vorstufe in einer wässrigen Dispersion vorliegt.

7. Verwendung nach Anspruch 6, **dadurch gekennzeichnet, dass** das Ubichinon Qₙ oder die Ubichinon Qₙ-Vorstufe in einer wässrigen kolloidalen Dispersion vorliegt.

## Claims

1. Use of ubiquinone Qₙ or ubiquinone Qₙ precursors for preparing a medicament for the treatment of pain caused by migraine.

2. The use according to claim 1, **characterized in that** said ubiquinone Qₙ is ubiquinone Q₁₀.

3. The use according to any of claims 1 to 2, **characterized in that** the treatment is done by administration in oral, parenteral, local, inhalative or intranasal form.

4. The use according to claim 3, **characterized in that** the administration is in the form of a spray.

5. The use according to claim 4, **characterized in that** the administration is in the form of a nasal spray.

6. The use according to any of claims 1 to 5, **characterized in that** said ubiquinone Qₙ or ubiquinone Qₙ precursor is in an aqueous dispersion.

7. The use according to claim 6, **characterized in that** said ubiquinone Qₙ or ubiquinone Qₙ precursor is in an aqueous colloidal dispersion.

## Revendications

1. Utilisation d'ubiquinone Qₙ ou de précurseurs d'ubiquinone Qₙ pour préparer des médicaments destinés au traitement des douleurs provoquées par des migraines.

2. Utilisation selon la revendication 1, **caractérisée en ce que** l'ubiquinone Qₙ est de l'ubiquinone Q₁₀.

3. Utilisation selon une des revendications 1 à 2, **caractérisée en ce que** le traitement s'effectue par administration sous une forme orale, parentérale, locale, inhalatrice ou intranasale.

4. Utilisation selon la revendication 3, **caractérisée en ce que** l'administration s'effectue sous forme d'un spray.

5. Utilisation selon la revendication 4, **caractérisée en ce que** l'administration s'effectue sous forme d'un spray nasal.

6. Utilisation selon une des revendications 1 à 5, **caractérisée en ce que** l'ubiquinone Qₙ ou les précurseurs d'ubiquinone Qₙ sont contenus dans une dispersion aqueuse.

7. Utilisation selon la revendication 6, **caractérisée en ce que** l'ubiquinone Qₙ ou les précurseurs d'ubiquinone Qₙ sont contenus dans une dispersion aqueuse colloïdale.
